# EUROPEAN PATENT SPECIFICATION

(11) **EP 4 424 847 B1**
(45) Date of publication and mention of the grant of the patent: **18.03.2026**
(21) Application number: 23892743.8
(22) Date of filing: 25.05.2023
(51) Int. Cl.: C13K 13/00, C12P 19/02, A23L 5/47

(54) **SYSTEM AND METHOD FOR CO-PRODUCTION OF PREMIUM-GRADE XYLOSE AND HIGH-END CARAMEL PIGMENT USING CORNCOBS**
SYSTEM UND VERFAHREN ZUR GLEICHZEITIGEN HERSTELLUNG VON XYLOSE MIT HOHEM ENDE UND KARAMELLPIGMENT MIT MAISSPINDELN
SYSTÈME ET PROCÉDÉ DE CO-PRODUCTION DE XYLOSE DE QUALITÉ SUPÉRIEURE ET DE PIGMENT DE CARAMEL HAUT DE GAMME À L'AIDE D'ÉPIS DE MAÏS

(30) Priority: 05.12.2022 CN 202211552492
(43) Date of publication of application: 04.09.2024
(73) Proprietor: Zhejiang Huakang Pharmaceutical Co., Ltd., Quzhou, Zhejiang 324302 (CN)
(72) Inventor: XU, Weidong, Quzhou, Zhejiang 324302 (CN); LI, Mian, Santa Clara, CA (US); YANG, Wulong, Quzhou, Zhejiang 324302 (CN); WU, Qiang, Quzhou, Zhejiang 324302 (CN); QIN, Shufang, Quzhou, Zhejiang 324302 (CN); XU, Jingjing, Quzhou, Zhejiang 324302 (CN); YAO, Zhihui, Quzhou, Zhejiang 324302 (CN)
(74) Representative: Sun, Yiming
(86) International application number: PCT/CN2023/096361
(87) International publication number: WO 2024/119730

(56) References cited:
- CN-A- 1 594 442
- CN-A- 101 220 381
- CN-A- 105 039 458
- CN-A- 105 713 998
- CN-A- 106 282 427
- CN-A- 114 213 215
- CN-A- 114 262 352
- CN-A- 116 083 657
- CN-U- 216 614 473
- CN-U- 218 860 763
- WANG QI ET AL: "Sugar Product Diversification and Its Opportunities in China", SUGAR TECH, SPRINGER INDIA, NEW DELHI, vol. 24, no. 4, 11 April 2022 (2022-04-11), pages 1093 - 1106, XP037908019, ISSN: 0972-1525, [retrieved on 20220411], DOI: 10.1007/S12355-022-01134-2

## Description

### TECHNICAL FIELD

The present invention discloses a system and a method for utilizing corn cobs to jointly produce a premium xylose and a high-end caramel pigment.

### BACKGROUND

Xylose is a type of pentose sugar, with a white crystal appearance and a sweet taste, equivalent to 0.7 times the sweetness of sucrose, which can be used as a sucrose substitute for sugar reduction and sweetening purposes. When compared to qualified xylose, premium xylose has a higher purity and light transmittance. High-end caramel pigments refer to those with a high color intensity and high durability, with more than twice the color intensity of regular caramel pigments. Chinese patent CN111647694A discloses a method for extracting xylose from corn cobs, involving processes such as acid hydrolysis, decolorization, separation, evaporation, and concentration, as well as crystallization and separation, resulting in a finished xylose product. Most of the solid residues of corn cob waste after acid hydrolysis are usually directly incinerated, while the majority of the xylose mother liquid after crystallization is often sold at a low price to syrup manufacturers, leading to significant waste of hexose sugars in the corn cob waste residues and pentose sugars in the xylose mother liquid.

CN216614473U discloses a utility model relating to a system for co-producing xylitol and caramel pigment by using xylose mother liquor. According to the utility model, the caramel pigment is prepared by purifying and crystallizing xylose from the xylose mother liquor and then preparing xylitol and centrifuged xylose chromatographic separation raffinate, so that the utilization value of the xylose mother liquor is improved.

CN105713998A discloses a production technique of xylose. The production technique comprises the following steps: pulverizing, boiling with high-temperature water, hydrolyzing, decolorizing, refining, carrying out continuous film concentration, carrying out steam vaporization concentration, crystallizing, centrifuging and drying, thereby finally obtaining the crystalline xylose finished product. The xylose mother solution is sold as the byproduct and used as the raw material for preparing the caramel pigment product. The corn cobs are pulverized, thereby increasing the specific area, shortening the contact and soak time, enhancing the hydrolysis efficiency, and increasing the xylose yield. The hydrochloric acid is used for hydrolyzing the corn cobs to prepare the xylose, thereby saving the subsequent decolorant consumption, and reducing the environmental pollution. The continuous film concentration technique is adopted to partially substitute the steam vaporization technique, thereby having favorable energy-saving effect.

CN101220381A provides a xylitol production method by utilizing corn cobs or agro-forestry waste, such as, stalks, as raw materials. The method includes the pretreatment of the raw materials, the diluted acid treatment of the raw materials and the solid-liquid separation, the ingredients of obtained liquid phase are treated with decolorization, filtration, deacidification and RO concentration, then a yeast is utilized for fermentation so as to obtain a xylitol. A relatively pure xylitol liquid is obtained after the bacteria removal, the concentration and the chromatographic separation of the fermentation liquid, then a solid finished product xylitol is obtained by decolorization, ash removal, concentration, crystallization, separation and drying, and a crystal parent solution returns to the chromatographic separation. In addition, the invention carries out the pulp conditioning of solid phase components which are obtained by the solid-liquid separation after acidolysis, then the solid phase components are used for ethanol fermentation after the enzymolysis; the lignin residue which is obtained after the filtration of the products of the enzymolysis can be taken as fuel or be used for the development of the deep-processed products of the lignin.

### SUMMARY

The present invention provides a system and a method for utilizing corn cobs to jointly produce a premium xylose and a high-end caramel pigment. The system uses corn cobs as a raw material to extract pentose sugar for the preparation of premium xylose, and processes pentose sugar and hexose sugar from a corn cob residue to produce the high-end caramel pigment. This approach not only reduces the waste of various sugar resources in corn cobs but also converts environmentally harmful agricultural waste into high-value products, thereby promoting high-end manufacturing, environmental and ecological protection, and high-quality economic development. The invention is set out in the appended set of claims.

### BRIEF DESCRIPTION OF THE DRAWINGS

FIG. 1 is a schematic diagram illustrating a principle of a system for utilizing corn cobs to jointly produce a premium xylose and a high-end caramel pigment according to some embodiments of the present disclosure; and
FIG. 2 is a schematic diagram illustrating a flow direction of each material in a method for utilizing corn cobs to jointly produce a premium xylose and a high-end caramel pigment according to some embodiments of the present disclosure.

### DETAILED DESCRIPTION

In order to make the technical problems, technical solutions, and beneficial effects to be solved by some embodiments of the present disclosure clearer and more understandable, the following combines the accompanying drawings and the embodiments to further explain some embodiments of the present disclosure in detail. It should be understood that the specific embodiments described herein are only for explaining some embodiments of the present disclosure and are not intended to limit some embodiments of the present disclosure.

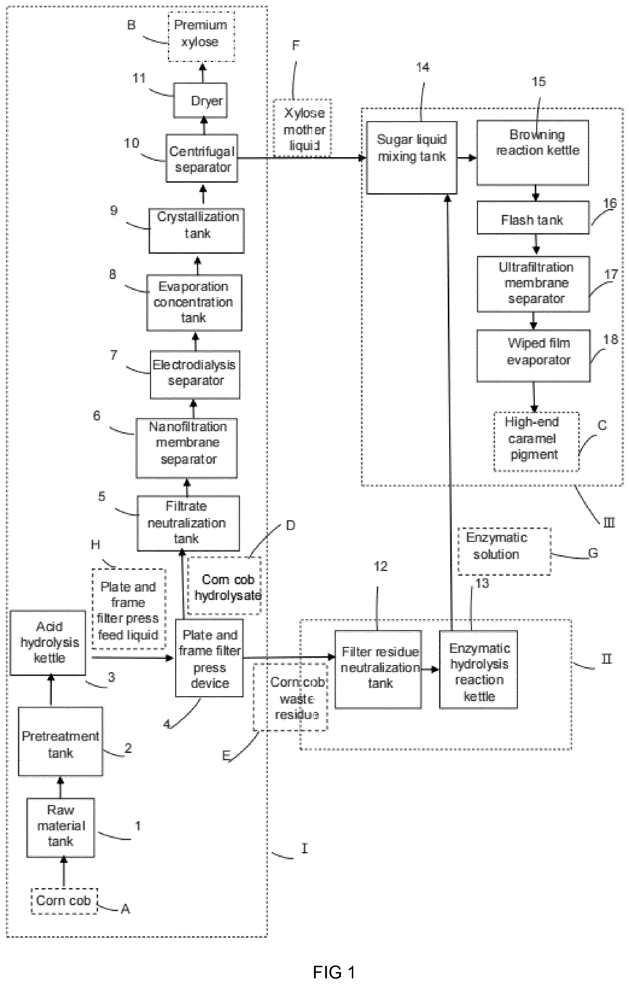

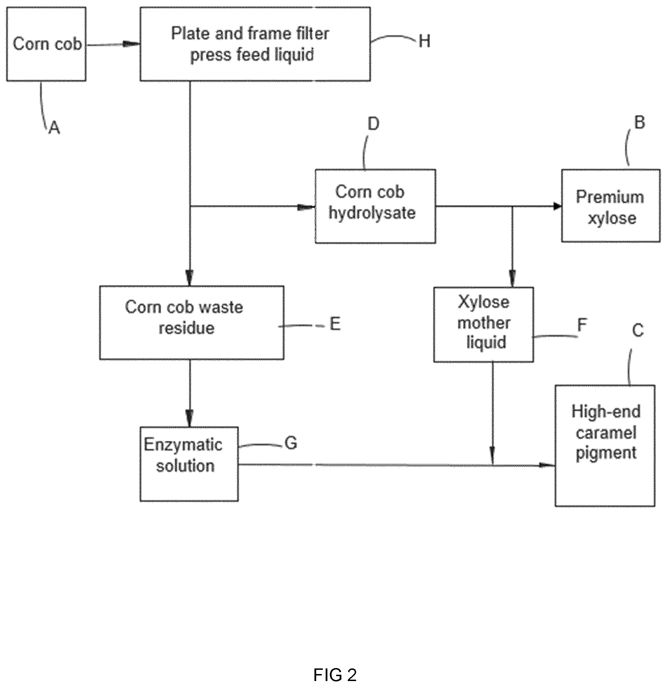

Please also refer to FIG. 1 and FIG. 2, a system for utilizing corn cobs to jointly produce a premium xylose and a high-end caramel pigment in some embodiments of the present disclosure includes a xylose preparation subsystem I, an enzymatic hydrolysis subsystem II, and a caramel pigment preparation subsystem III that are connected to each other by pipelines. The directions of the arrows in the figures illustrate the directions of the flow of materials in the system.

The xylose preparation subsystem I includes a raw material tank 1, a pretreatment tank 2, an acid hydrolysis kettle 3, a plate and frame filter press device 4, a filtrate neutralization tank 5, a nanofiltration membrane separator 6, an electrodialysis separator 7, an evaporation concentration tank 8, a crystallization tank 9, a centrifugal separator 10, and a dryer 11, which are connected sequentially by pipelines.

The enzymatic hydrolysis subsystem II includes a filter residue neutralization tank 12 and an enzymatic hydrolysis reaction kettle 13 connected by a pipeline.

The caramel pigment preparation subsystem III includes a sugar liquid mixing tank 14, a browning reaction kettle 15, a flash tank 16, an ultrafiltration membrane separator 17, and a wiped film evaporator 18 connected sequentially by pipelines.

The raw material tank 1 stores a raw material to be processed of a corn cob A, a material outputted from a discharge port of the dryer 11 is a premium xylose B, and a material finally obtained from a discharge port of the wiped film evaporator 18 is a high-end caramel pigment C.

The plate and frame filter press device 4 is provided with a filtrate outlet and a filter residue outlet, the filtrate outlet is connected to the filtrate neutralization tank 5 through a pipeline, and the filter residue outlet is connected to a feed port of the filter residue neutralization tank 12 through a pipeline. The filtrate output from the filtrate outlet of the plate and frame filter press device 4 may be a corn cob hydrolysate D, and the filter residue output from the filter residue outlet of the plate and frame filter press device 4 may be a corn cob waste residue E.

The centrifugal separator 10 is provided with a solid outlet and a liquid outlet, respectively. The solid outlet is connected to a feed port of the dryer 11 via a pipeline, and the liquid outlet is connected to one feed port of the sugar liquid mixing tank 14. The other feed port of the sugar liquid mixing tank 14 is connected to a discharge port of the enzymatic hydrolysis reaction kettle 13 through a pipeline.

In some embodiments, a liquid material transported from a liquid outlet of the centrifugal separator 10 may be a xylose mother liquid F. The liquid output from the discharge port of the enzymatic hydrolysis reaction kettle 13 may be an enzymatic solution G.

In some embodiments, the pretreatment tank 2 may be used to wash away impurities in the corn cob A using water or dilute acid, the acid hydrolysis kettle 3 may be used to hydrolyze hemicellulose in the corn cob A, and the plate and frame filter press device 4 may be used to separate the corn cob hydrolysate D from the corn cob waste residue E. The filtrate neutralization tank 5 may be used to neutralize the pH of the corn cob hydrolysate D. The nanofiltration membrane separator 6 may be used for decolorization of the corn cob hydrolysate D. The retentate after decolorization treatment of the nanofiltration membrane separator 6 may be a pigmented liquid, and the permeate may be a decolorized liquid.

The electrodialysis separator 7 may be used for desalination of a decolorized solution. In some embodiments, the retentate after desalination treatment of the electrodialysis separator 7 may be a concentrated saline solution, and the permeate may be a desalinated solution. The evaporation concentration tank 8 and the crystallization tank 9 may be used for evaporation and concentration, and xylose crystallization. The centrifugal separator 10 may be used for separating a crystallized xylose and the xylose mother liquid F, and the dryer 11 may be used for drying a crystal of the premium xylose B.

The filter residue neutralization tank 12 may be used to neutralize the pH of the corn cob waste residue E. The enzymatic hydrolysis reaction kettle 13 may be used to enzymatically hydrolyze the cellulose in the corn cob waste residue E into glucose. The sugar liquid mixing tank 14 may be used for mixing the xylose mother liquid F and the enzymatic solution G to obtain a mixed sugar solution. The browning reaction kettle 15 may be used for browning reaction of the mixed sugar solution to produce a crude high-end caramel pigment. The flash tank 16 may be used to remove impurities such as amino compounds or sulfites in the caramel pigment. The ultrafiltration membrane separator 17 may be used to remove impurities such as 4-methylimidazole in the caramel pigment, and the wiped film evaporator 18 may be used for low-temperature concentration of the caramel pigment to obtain the high-end caramel pigment C. The present invention further discloses a method for utilizing corn cobs to jointly produce a premium xylose and a high-end caramel pigment, the method using the system for utilizing corn cobs to jointly produce a premium xylose and a high-end caramel pigment. The method includes:
Step 1, transporting corn cob powder stored in the raw material tank to the pretreatment tank through a pipeline for water washing and acid washing to obtain a corn cob liquid, and then sending the corn cob liquid to the acid hydrolysis kettle through a pipeline to perform acid hydrolysis treatment;
Step 2, obtaining a plate and frame filter press feed liquid H after the acid hydrolysis treatment, and sending the plate and frame filter press feed liquid H to the plate and frame filter press device 4 through a pipeline for filtration treatment;
Step 3, filtering the plate and frame filter press feed liquid H by the plate and frame filter press device 4 to obtain a liquid part and a solid part, respectively; wherein the liquid part is the corn cob hydrolysate D, and the solid part is the corn cob waste residue E;
Step 4, performing neutralization treatment of the filtrate neutralization tank 5, decolorization treatment of the nanofiltration membrane separator 6, desalination treatment of the electrodialysis separator 7, concentration treatment of the evaporation concentration tank 8, crystallization treatment of the crystallization tank 9, and solid-liquid separation treatment of the centrifugal separator 10 on the corn cob hydrolysate D to obtain a crystallized xylose and a xylose mother liquid, performing drying treatment of the dryer 11 on the crystallized xylose to obtain a crystal finished product of the premium xylose B, and transporting the xylose mother liquid F to the sugar liquid mixing tank 14 through a pipeline;
the purity of the crystal of the premium xylose B is larger than 99%, and a light transmittance is larger than 98%; and
Step 5, transporting the corn cob waste residue E sequentially to the filter residue neutralization tank 12 for neutralization treatment and to the enzymatic hydrolysis reaction kettle 13 for enzymatic hydrolysis treatment to obtain the enzymatic solution G, transporting the enzymatic solution G to the sugar liquid mixing tank 14 through a pipeline to be mixed with the xylose mother liquid F to obtain a mixture solution, and then transporting the mixture solution through browning reaction treatment of the browning reaction kettle 15, evaporation treatment of the flash tank 16, separation treatment of the ultrafiltration membrane separator 17, and wiped film evaporation treatment of the wiped film evaporator 18 to obtain a product of the high-end caramel pigment C; wherein a color ratio of the high-end caramel pigment C is as high as 50000 EBC.

In some embodiments, in Step 1, a mass percentage concentration of dry sugar resources in the corn cob A may be in a range of 60-70 wt%, wherein the dry sugar resources may contain hexose sugars of 50~55wt% and pentose sugars of 45~50wt %, a sulfuric acid solution of 0.20~0.30 wt% may be used for the acid washing, and a liquid-to-solid ratio for the acid washing may be 3:1.

In some embodiments, in Step 2, during the acid hydrolysis treatment, a ratio of corn cob material to liquid may be 1:5, an added sulfuric acid content may be in a range of 1.2-1.5%, a temperature may be in a range of 120~125 °C, and a time may be in a range of 100-140 min.

In some embodiments, in Step 4, during the decolorization treatment of the nanofiltration membrane separator 6, an operating temperature of the nanofiltration membrane separator 6 may be in a range of 40~60°C, and an operating pressure may be in a range of 15~35 bar; during the desalination treatment of the electrodialysis separator 7, an operating current of the electrodialysis separator 7 may be in a range of 60-120 A, a voltage may be in a range of 200-250 V, and an operating pressure may be in a range of 15~35 bar.

In some embodiments, in Step 5, during the enzymatic hydrolysis treatment, the pH may be adjusted to 5.5-6.5, a commercial cellulose composite enzyme with a percentage of 2~4% may be used as a catalyst, an enzymatic reaction temperature may be controlled in a range of 40-60 °C, and an enzymatic reaction time may be in a range of 72-96 h; during the browning reaction treatment of the browning reaction kettle 15, a reaction auxiliary agent may be a compound of ammonia water or ammonium carbonate, ammonium bicarbonate, and urea, a compound ratio may be in a range of 6:2:2-4:4:2. A reaction process may include: first adjusting the pH to 7.0-9.0, then raising the temperature to 80-90 °C to react for 30-50 min, then raising the temperature to 160-180 °C for 3~5 h, then adjusting the pH to 2-4, then lowering the temperature to 130-150 °C to react for 0.5~1 h. During the separation treatment of the ultrafiltration membrane separator 17, an operating temperature of the ultrafiltration membrane separator 17 may be in a range of 40-60 °C, and an operating pressure may be in a range of 5~7 bar; and during wiped film evaporation treatment of the wiped film evaporator 18, an evaporation pressure may be in a range of 4~5bar, and a vacuum degree may be in a range of 80~ 90 kpa.

The system and method of utilizing corn cobs to jointly produce a premium xylose and a high-end caramel pigment of the present disclosure will be further described below through specific examples.

### Example1

In processes for utilizing corn cobs to jointly produce a premium xylose and a high-end caramel pigment using the corn cobs as a raw material, after purifying the premium xylose, high-end caramel pigments may be prepared by using an enzymatic solution after enzymatic hydrolysis and mixing with a xylose mother liquid after centrifugation according to following steps:
Step 1, transporting the corn cob powder stored in the raw material tank 1 to the pretreatment tank through a pipeline for water washing and acid washing to obtain a corn cob liquid, using a sulfuric acid solution of 0.20 wt% with a liquid-to-solid ratio of 3:1 for the acid washing, then sending the corn cob liquid to the acid hydrolysis kettle 3 to perform acid hydrolysis treatment;
Step 2, transporting a post-wash liquid of the corn cobs in the pretreatment tank 2 to the acid hydrolysis kettle 3 through a pipeline for hydrolysis treatment, with a corn cob liquid-to-solid ratio of 5:1, an added sulfuric acid content of 1.2%, a temperature of 125 °C, and a time of 140 min, then sending a hydrolyzed treated material to the plate and frame filter press device 4 for filtration treatment;
Step 3, dividing the hydrolyzed treated material into two parts by the plate and frame filter press device 4, a liquid part being the corn cob hydrolysate D, and a solid part being the corn cob waste residue E;
Step 4, performing neutralization treatment of the filtrate neutralization tank 5, decolorization treatment of the nanofiltration membrane separator 6, desalination treatment of the electrodialysis separator 7, concentration treatment of the evaporation concentration tank 8, crystallization treatment of the crystallization tank 9, and solid-liquid separation treatment of the centrifugal separator 10 on the corn cob hydrolysate D to obtain a crystallized xylose and a xylose mother liquid F, performing drying treatment of the dryer 11 on the crystallized xylose to obtain a crystal finished product of the premium xylose B, transporting the xylose mother liquid F to the sugar liquid mixing tank 14 through a pipeline;
the purity of the crystal of the premium xylose may be larger than 99%, and a light transmittance may be larger than 98%; the operating temperature of the nanofiltration membrane separator 6 may be 60 °C, and the operating pressure may be 20 bar; and the operating current of the electrodialysis separator 7 may be 100 A, the voltage may be 250 V, and the operating pressure may be 20 bar;
Step 5, transporting the corn cob waste residue sequentially to the filter residue neutralization tank 12 for neutralization treatment and to the enzymatic hydrolysis reaction kettle 13 for enzymatic hydrolysis treatment to obtain the enzymatic solution G, transporting the enzymatic solution G to the sugar liquid mixing tank 14 through a pipeline to be mixed with the xylose mother liquid F to obtain a mixture solution, and then transporting the mixture solution through the browning reaction treatment of the browning reaction kettle 15, the evaporation treatment of the flash tank 16, the separation treatment of the ultrafiltration membrane separator 17, and the wiped film evaporation treatment of the wiped film evaporator 18 to obtain a product of the high-end caramel pigment C, wherein the color ratio of the high-end caramel pigment C is as high as 50,000 EBC, all other indexes are all compounded with the national standard, and a utilization rate of sugar resources is as high as 90%.

In some embodiments, during the enzymatic hydrolysis treatment, the pH may be adjusted to 5.5, and a commercial cellulose composite enzyme with a percentage of 2% may be added as a catalyst, the enzymatic reaction temperature may be controlled at 50 °C, and the enzymatic reaction time may be 72 h.

In some embodiments, during the browning reaction treatment of the browning reaction kettle 15, the reaction auxiliary agent may be a compound of ammonia water or ammonium carbonate, ammonium bicarbonate, and urea, a compound ratio may be 6:2:2. The reaction process may include: first adjusting the pH to 9.0, and then raising the temperature to 90 °C to react for 30 min, then raising the temperature to 170 °C to react for 3 h, then adjusting the pH to 3, then lowering the temperature to 130 °C to react for 1 h. The operating temperature of the ultrafiltration membrane separator 17 may be 60 °C, and an operating pressure may be 5 bar. During the wiped film evaporation treatment of the wiped film evaporator 18, the evaporation pressure may be 4 bar, and the vacuum degree may be 90 kpa.

### Comparative Example1

The corn cob A was used as a raw material for the direct extraction of xylose. The extraction process includes: transporting the raw material of the corn cob in the raw material tank 1 through pipelines to the pretreatment tank 2 for washing, transporting the washed raw material to the acid hydrolysis kettle 3, adding 1.5% sulfuric acid, and then performing alkali neutralization, activated carbon decolorization, ion exchange desalination by passing the plate and frame filter press device 4 on the hydrolysate, and then performing evaporation concentration, crystallization, and centrifugation to finally obtain a finished product crystallized xylose. After the plate and frame press filtration, the corn cob waste residue E was dried and burned or treated as solid waste, and the xylose mother liquid F after centrifugation was sold as syrup, and the actual utilization rate of sugar resources was only 30%.

### Comparative Example 2

The caramel pigment was directly prepared using the xylose mother liquid F as a raw material, and the specific process include: transporting the xylose mother liquid F after centrifugal separation directly to the browning reaction kettle 15 through a pipeline, adjusting the pH to 9.0, then adding 10w% ammonium carbonate as a reaction aid, wherein the reaction temperature was 170 °C, and the reaction time was 3 hours. After the reaction, the finished product of caramel pigment was obtained by filtration. The color ratio of the caramel pigment was 30,000 EBC, and all other indexes were in accordance with the national standard.

In summary, using the system and method for utilizing corn cobs to jointly produce a premium xylose and a high-end caramel pigment of some embodiments of the present disclosure, the utilization rate of the sugar resources in the corn cob A is increased from 30% to 90%, and the color ratio of the caramel pigment is increased from 30,000 EBC to 50,000 EBC, which improves the comprehensive utilization value of the corn cob and also improves the product quality of the caramel pigment.

## Claims

1. A system for utilizing corn cobs to jointly produce a premium xylose and a high-end caramel pigment, comprising a xylose preparation subsystem, an enzymatic hydrolysis subsystem, and a caramel pigment preparation subsystem that are connected to each other through pipelines; wherein
the xylose preparation subsystem includes a raw material tank, a pretreatment tank, an acid hydrolysis kettle, a plate and frame filter press device, a filtrate neutralization tank, a nanofiltration membrane separator, an electrodialysis separator, an evaporation concentration tank, a crystallization tank, a centrifugal separator, and a dryer that are connected in sequence through pipelines;
the enzymatic hydrolysis subsystem includes a filter residue neutralization tank and an enzymatic hydrolysis reaction kettle connected through pipelines;
the caramel pigment preparation subsystem includes a sugar liquid mixing tank, a browning reaction kettle, a flash tank, an ultrafiltration membrane separator, and a wiped film evaporator connected in sequence through pipelines; and
the plate and frame filter press device is provided with a filtrate outlet and a filter residue outlet; the filtrate outlet is connected to the filtrate neutralization tank through a pipeline; the filter residue outlet is connected to a feed port of the filter residue neutralization tank through a pipeline, the centrifugal separator is provided with a solid outlet and a liquid outlet, the solid outlet is connected to a feed port of the dryer through a pipeline, and the liquid outlet is connected to one feed port of the sugar liquid mixing tank, the other feed port of the sugar liquid mixing tank is connected to a discharge port of the enzymatic hydrolysis reaction kettle through a pipeline, the raw material tank stores a raw material to be processed of the corn cobs, a material output by a discharge port of the dryer is the premium xylose, and a final material obtained from a discharge port of the wiped film evaporator is the high-end caramel pigment.

2. The system of claim 1, wherein a filtrate output from a filtrate outlet of the plate and frame filter press device is a corn cob hydrolysate, and a filter residue output from a filter residue outlet of the plate and frame filter press device is a corn cob waste residue.

3. The system of claim 1, wherein a liquid material transported from a liquid outlet of the centrifugal separator is a xylose mother liquid.

4. The system of claim 1, wherein a liquid output from the discharge port of the enzymatic hydrolysis reaction kettle is an enzymatic solution.

5. A method for utilizing corn cobs to jointly produce a premium xylose and a high-end caramel pigment using the system of any one of claims 1-4, comprising:
Step 1: transporting corn cob powder stored in the raw material tank to the pretreatment tank through a pipeline for water washing and acid washing to obtain a corn cob liquid, and then sending the corn cob liquid to the acid hydrolysis kettle through a pipeline to perform acid hydrolysis treatment;
Step 2: obtaining a plate and frame filter press feed liquid after the acid hydrolysis treatment, and sending the plate and frame filter press feed liquid to the plate and frame filter press device through a pipeline for filtration treatment;
Step 3: filtering the plate and frame filter press feed liquid by the plate and frame filter press device to obtain a liquid part and a solid part, respectively; wherein the liquid part is a corn cob hydrolysate, and the solid part is a corn cob waste residue;
Step 4: performing neutralization treatment of the filtrate neutralization tank, decolorization treatment of the nanofiltration membrane separator, desalination treatment of the electrodialysis separator, concentration treatment of the evaporation concentration tank, crystallization treatment of the crystallization tank, and solid-liquid separation treatment of the centrifugal separator on the corn cob hydrolysate to obtain a crystallized xylose and a xylose mother liquid, respectively, performing drying treatment of the dryer on the crystallized xylose to obtain a crystal finished product of a premium xylose, transporting the xylose mother liquid to the sugar liquid mixing tank through a pipeline; wherein a purity of the crystal finished product of the premium xylose is larger than 99%, and a light transmittance of the crystal finished product of the premium xylose is larger than 98%; and
Step 5: transporting the corn cob waste residue sequentially to the filter residue neutralization tank for neutralization treatment and to the enzymatic hydrolysis reaction kettle for enzymatic hydrolysis treatment to obtain an enzymatic solution, transporting the enzymatic solution to the sugar liquid mixing tank through a pipeline to be mixed with the xylose mother liquid to obtain a mixture solution, and then transporting the mixture solution through browning reaction treatment of the browning reaction kettle, evaporation treatment of the flash tank, separation treatment of the ultrafiltration membrane separator, and wiped film evaporation treatment of the wiped film evaporator to obtain a product of the high-end caramel pigment; wherein a color ratio of the high-end caramel pigment is as high as 50000 EBC.

6. The method of claim 5, wherein in Step 1, a mass percentage concentration of dry sugar resources in the corn cobs is in a range of 60-70 wt%, the dry sugar resources contain hexose sugars of 50-55 wt% and pentose sugars of 45-50 wt%; a sulfuric acid solution of 0.20~0.30 wt% is used for the acid washing, and a liquid-to-solid ratio for the acid washing is 3:1.

7. The method of claim 5, wherein in Step 2, during the acid hydrolysis treatment, a ratio of corn cob material to liquid is 1:5, an added sulfuric acid content is in a range of 1.2-1.5%, a temperature is in a range of 120-125 °C, and a time is in a range of 100-140 min.

8. The method of claim 5, wherein in Step 4, during the decolorization treatment of the nanofiltration membrane separator, an operating temperature of the nanofiltration membrane separator is in a range of 40-60 °C, and an operating pressure is in a range of 15~35 bar; and during the desalination treatment of the electrodialysis separator, an operating current of the electrodialysis separator is in a range of 60-120 A, a voltage is in a range of 200-250 V, and an operating pressure is in a range of 15-35 bar.

9. The method of claim 5, wherein in Step 5, during the enzymatic hydrolysis treatment, pH is adjusted to 5.5-6.5, and a commercial cellulose composite enzyme with a percentage of 2~4 % is added as a catalyst, an enzymatic hydrolysis reaction temperature is controlled in a range of 40-60 °C, and an enzymatic hydrolysis reaction time is in a range of 72-96 h; during the browning reaction treatment of the browning reaction kettle, a reaction auxiliary agent is a compound of ammonia water or ammonium carbonate, ammonium bicarbonate, and urea, a compound ratio is in a range of 6:2:2-4:4:2,
a reaction process includes: first adjusting pH to 7.0-9.0, and then raising the temperature to 80-90 °C to react for 30-50 min, then raising the temperature to 160-180 °C to react for 3~5 h, then adjusting the pH to 2-4, and then lowering the temperature to 130-150 °C to react for 0.5~1 h;
during the separation treatment of the ultrafiltration membrane separator, an operating temperature of the ultrafiltration membrane separator is in a range of 40-60 °C, and an operating pressure is in a range of 5~7 bar; and
during the wiped film evaporation treatment of the wiped film evaporator, an evaporation pressure is in a range of 4~5 bar, and a vacuum degree is in a range of 80~ 90 kpa.

## Patentansprüche

1. Ein System zur Nutzung von Maiskolben zur gemeinsamen Herstellung einer Premium-Xylose und eines High-End-Karamellfarbstoffs, umfassend ein Xylose-Herstellungsteilsystem, ein enzymatisches Hydrolyseteilsystem und ein Karamellfarbstoff-Herstellungsteilsystem, die über Rohrleitungen miteinander verbunden sind; wobei
das Xylose-Herstellungsteilsystem einen Rohstoffbehälter, einen Vorbehandlungsbehälter, einen Säurehydrolysekessel, eine Platten- und Rahmenfilterpressvorrichtung, einen Filtratneutralisationsbehälter, einen Nanofiltrationsmembran-Trenner, einen Elektrodialyse-Trenner, einen Eindampf-Konzentrationsbehälter, einen Kristallisationsbehälter, eine Zentrifuge und einen Trockner umfasst, die der Reihe nach über Rohrleitungen verbunden sind;
das enzymatische Hydrolyseteilsystem einen Filterrückstandsneutralisationsbehälter und einen enzymatischen Hydrolysereaktionskessel umfasst, die über Rohrleitungen verbunden sind;
das Karamellfarbstoff-Herstellungsteilsystem einen Zuckerlösungsmischbehälter, einen Bräunungsreaktionskessel, einen Flashbehälter, einen Ultrafiltrationsmembran-Trenner und einen Wischfilmverdampfer umfasst, die der Reihe nach über Rohrleitungen verbunden sind; und
die Platten- und Rahmenfilterpressvorrichtung mit einem Filtratauslass und einem Filterrückstandsauslass versehen ist; der Filtratauslass ist über eine Rohrleitung mit dem Filtratneutralisationsbehälter verbunden; der Filterrückstandsauslass ist über eine Rohrleitung mit einer Zuführöffnung des Filterrückstandsneutralisationsbehälters verbunden, die Zentrifuge mit einem Feststoffauslass und einem Flüssigkeitsauslass versehen ist, der Feststoffauslass ist über eine Rohrleitung mit einer Zuführöffnung des Trockners verbunden, und der Flüssigkeitsauslass ist mit einer Zuführöffnung des Zuckerlösungsmischbehälters verbunden, eine andere Zuführöffnung des Zuckerlösungsmischbehälters ist über eine Rohrleitung mit einer Austragsöffnung des enzymatischen Hydrolysereaktionskessels verbunden, der Rohstoffbehälter ein zu verarbeitendes Rohmaterial der Maiskolben speichert, ein Material, das durch eine Austragsöffnung des Trockners ausgegeben wird, die Premium-Xylose ist, und ein Endmaterial, das aus einer Austragsöffnung des Wischfilmverdampfers erhalten wird, der High-End-Karamellfarbstoff ist.

2. Das System nach Anspruch 1, wobei ein Filtrat, das aus einem Filtratauslass der Platten- und Rahmenfilterpressvorrichtung ausgegeben wird, ein Maiskolbenhydrolysat ist, und ein Filterrückstand, der aus einem Filterrückstandsauslass der Platten- und Rahmenfilterpressvorrichtung ausgegeben wird, ein Maiskolbenabfallrückstand ist.

3. Das System nach Anspruch 1, wobei ein Flüssigkeitsmaterial, das von einem Flüssigkeitsauslass der Zentrifuge gefördert wird, eine Xylose-Mutterlauge ist.

4. Das System nach Anspruch 1, wobei eine Flüssigkeit, die aus der Austragsöffnung des enzymatischen Hydrolysereaktionskessels ausgegeben wird, eine Enzymlösung ist.

5. Ein Verfahren zur Nutzung von Maiskolben zur gemeinsamen Herstellung einer Premium-Xylose und eines High-End-Karamellfarbstoffs unter Verwendung des Systems nach einem der Ansprüche 1-4, umfassend:
Schritt 1: Überführen von im Rohstoffbehälter gespeichertem Maiskolbenpulver über eine Rohrleitung in den Vorbehandlungsbehälter zur Wasserwäsche und Säurewäsche, um eine Maiskolbenflüssigkeit zu erhalten, und anschließendes Überführen der Maiskolbenflüssigkeit über eine Rohrleitung in den Säurehydrolysekessel, um eine Säurehydrolysebehandlung durchzuführen;
Schritt 2: Erhalten einer Zufuhrflüssigkeit für die Platten- und Rahmenfilterpresse nach der Säurehydrolysebehandlung und Überführen der Zufuhrflüssigkeit für die Platten- und Rahmenfilterpresse über eine Rohrleitung in die Platten- und Rahmenfilterpressvorrichtung zur Filtrationsbehandlung;
Schritt 3: Filtrieren der Zufuhrflüssigkeit für die Platten- und Rahmenfilterpresse durch die Platten- und Rahmenfilterpressvorrichtung, um jeweils einen Flüssigkeitsteil und einen Feststoffteil zu erhalten; wobei der Flüssigkeitsteil ein Maiskolbenhydrolysat ist und der Feststoffteil ein Maiskolbenabfallrückstand ist;
Schritt 4: Durchführen nacheinander, an dem Maiskolbenhydrolysat, einer Neutralisationsbehandlung mittels des Filtratneutralisationsbehälters, einer Entfärbungsbehandlung mittels des Nanofiltrationsmembran-Trenners, einer Entsalzungsbehandlung mittels des Elektrodialyse-Trenners, einer Konzentrationsbehandlung mittels des Eindampf-Konzentrationsbehälters, einer Kristallisationsbehandlung mittels des Kristallisationsbehälters und einer Fest-Flüssig-Trennbehandlung mittels der Zentrifuge, um jeweils kristallisierte Xylose und eine Xylose-Mutterlauge zu erhalten, Durchführen einer Trocknungsbehandlung des Trockners an der kristallisierten Xylose, um ein kristallines Endprodukt einer Premium-Xylose zu erhalten, und Überführen der Xylose-Mutterlauge über eine Rohrleitung in den Zuckerlösungsmischbehälter; wobei eine Reinheit des kristallinen Endprodukts der Premium-Xylose größer als 99% ist und eine Lichtdurchlässigkeit des kristallinen Endprodukts der Premium-Xylose größer als 98% ist; und
Schritt 5: Überführen des Maiskolbenabfallrückstands nacheinander in den Filterrückstandsneutralisationsbehälter zur Neutralisationsbehandlung und in den enzymatischen Hydrolysereaktionskessel zur enzymatischen Hydrolysebehandlung, um eine Enzymlösung zu erhalten, Überführen der Enzymlösung über eine Rohrleitung in den Zuckerlösungsmischbehälter, um mit der Xylose-Mutterlauge gemischt zu werden, um eine Mischlösung zu erhalten, und anschließendes Überführen der Mischlösung durch eine Bräunungsreaktionsbehandlung des Bräunungsreaktionskessels, eine Verdampfungsbehandlung des Flashbehälters, eine Trennbehandlung des Ultrafiltrationsmembran-Trenners und eine Wischfilmverdampfungsbehandlung des Wischfilmverdampfers, um ein Produkt des High-End-Karamellfarbstoffs zu erhalten; wobei ein Farbwert des High-End-Karamellfarbstoffs bis zu 50000 EBC beträgt.

6. Das Verfahren nach Anspruch 5, wobei in Schritt 1 eine Massenprozentkonzentration an trockenen Zuckerbestandteilen in den Maiskolben im Bereich von 60-70 wt% liegt, wobei die trockenen Zuckerbestandteile Hexosezucker von 50-55 wt% und Pentosezucker von 45-50 wt% enthalten; eine Schwefelsäurelösung von 0,20-0,30 wt% für die Säurewäsche verwendet wird, und ein Flüssig-Feststoff-Verhältnis für die Säurewäsche 3:1 beträgt.

7. Das Verfahren nach Anspruch 5, wobei in Schritt 2 während der Säurehydrolysebehandlung ein Verhältnis von Maiskolbenmaterial zu Flüssigkeit 1:5 beträgt, ein zugesetzter Schwefelsäuregehalt im Bereich von 1,2-1,5% liegt, eine Temperatur im Bereich von 120-125 °C liegt und eine Zeit im Bereich von 100-140 min liegt.

8. Das Verfahren nach Anspruch 5, wobei in Schritt 4 während der Entfärbungsbehandlung des Nanofiltrationsmembran-Trenners eine Betriebstemperatur des Nanofiltrationsmembran-Trenners im Bereich von 40-60 °C liegt und ein Betriebsdruck im Bereich von 15-35 bar liegt; und während der Entsalzungsbehandlung des Elektrodialyse-Trenners ein Betriebsstrom des Elektrodialyse-Trenners im Bereich von 60-120 A liegt, eine Spannung im Bereich von 200-250 V liegt und ein Betriebsdruck im Bereich von 15-35 bar liegt.

9. Das Verfahren nach Anspruch 5, wobei in Schritt 5 während der enzymatischen Hydrolysebehandlung der pH auf 5,5-6,5 eingestellt wird und ein kommerzielles Cellulose-Verbundenzym mit einem Anteil von 2~4 % als Katalysator zugesetzt wird, eine enzymatische Hydrolysereaktionstemperatur im Bereich von 40-60 °C gesteuert wird und eine enzymatische Hydrolysereaktionszeit im Bereich von 72-96 h liegt; während der Bräunungsreaktionsbehandlung des Bräunungsreaktionskessels ein Reaktionshilfsmittel eine Verbindung aus Ammoniakwasser oder Ammoniumcarbonat, Ammoniumhydrogencarbonat und Harnstoff ist, ein Verbindungsverhältnis im Bereich von 6:2:2-4:4:2 liegt,
ein Reaktionsprozess umfasst: zuerst Einstellen des pH auf 7,0-9,0 und dann Erhöhen der Temperatur auf 80-90 °C, um 30-50 min zu reagieren, dann Erhöhen der Temperatur auf 160-180 °C, um 3~5 h zu reagieren, dann Einstellen des pH auf 2~4 und dann Absenken der Temperatur auf 130-150 °C, um 0,5~1 h zu reagieren;
während der Trennbehandlung des Ultrafiltrationsmembran-Trenners eine Betriebstemperatur des Ultrafiltrationsmembran-Trenners im Bereich von 40-60 °C liegt und ein Betriebsdruck im Bereich von 5-7 bar liegt; und
während der Wischfilmverdampfungsbehandlung des Wischfilmverdampfers ein Verdampfungsdruck im Bereich von 4~5 bar liegt und ein Vakuumgrad im Bereich von 80~ 90 kpa liegt.

## Revendications

1. Un système d'utilisation de rafles de maïs pour coproduire un xylose premium et un pigment caramel haut de gamme, comprenant un sous-système de préparation du xylose, un sous-système d'hydrolyse enzymatique et un sous-système de préparation du pigment caramel, qui sont reliés entre eux par des conduites; dans lequel
le sous-système de préparation du xylose comprend un réservoir de matière première, une cuve de prétraitement, un réacteur d'hydrolyse acide, un filtre-presse à plaques et à cadres, une cuve de neutralisation du filtrat, un séparateur à membrane de nanofiltration, un séparateur d'électrodialyse, une cuve de concentration par évaporation, une cuve de cristallisation, un séparateur centrifuge et un sécheur, lesquels sont reliés en séquence par des conduites;
le sous-système d'hydrolyse enzymatique comprend une cuve de neutralisation des résidus de filtration et un réacteur d'hydrolyse enzymatique reliés par des conduites;
le sous-système de préparation du pigment caramel comprend une cuve de mélange de solution sucrée, un réacteur de brunissement, une cuve flash, un séparateur à membrane d'ultrafiltration et un évaporateur à film raclé reliés en séquence par des conduites; et
le filtre-presse à plaques et à cadres est pourvu d'une sortie de filtrat et d'une sortie de résidus de filtration; la sortie de filtrat est reliée à la cuve de neutralisation du filtrat par une conduite; la sortie de résidus de filtration est reliée à un orifice d'alimentation de la cuve de neutralisation des résidus de filtration par une conduite, le séparateur centrifuge est pourvu d'une sortie de solides et d'une sortie de liquide, la sortie de solides est reliée à un orifice d'alimentation du sécheur par une conduite, et la sortie de liquide est reliée à un orifice d'alimentation de la cuve de mélange de solution sucrée, un autre orifice d'alimentation de la cuve de mélange de solution sucrée est relié à un orifice de décharge du réacteur d'hydrolyse enzymatique par une conduite, le réservoir de matière première stocke une matière première à traiter des rafles de maïs, un matériau délivré par un orifice de décharge du sécheur est le xylose premium, et un matériau final obtenu à partir d'un orifice de décharge de l'évaporateur à film raclé est le pigment caramel haut de gamme.

2. Le système selon la revendication 1, dans lequel un filtrat délivré à partir d'une sortie de filtrat du filtre-presse à plaques et à cadres est un hydrolysat de rafles de maïs, et des résidus de filtration délivrés à partir d'une sortie de résidus de filtration du filtre-presse à plaques et à cadres sont des résidus usés de rafles de maïs.

3. Le système selon la revendication 1, dans lequel un liquide transporté depuis une sortie de liquide du séparateur centrifuge est une liqueur mère de xylose.

4. Le système selon la revendication 1, dans lequel un liquide délivré à partir de l'orifice de décharge du réacteur d'hydrolyse enzymatique est une solution enzymatique.

5. Un procédé d'utilisation de rafles de maïs pour coproduire un xylose premium et un pigment caramel haut de gamme en utilisant le système de l'une quelconque des revendications 1-4, comprenant:
Étape 1: transporter une poudre de rafles de maïs stockée dans le réservoir de matière première vers la cuve de prétraitement par une conduite pour un lavage à l'eau et un lavage acide afin d'obtenir un liquide de rafles de maïs, puis envoyer le liquide de rafles de maïs vers le réacteur d'hydrolyse acide par une conduite pour effectuer un traitement d'hydrolyse acide;
Étape 2: obtenir un liquide d'alimentation du filtre-presse à plaques et à cadres après le traitement d'hydrolyse acide, et envoyer le liquide d'alimentation du filtre-presse à plaques et à cadres vers le filtre-presse à plaques et à cadres par une conduite pour un traitement de filtration;
Étape 3: filtrer le liquide d'alimentation du filtre-presse à plaques et à cadres par le filtre-presse à plaques et à cadres afin d'obtenir respectivement une partie liquide et une partie solide; dans lequel la partie liquide est un hydrolysat de rafles de maïs, et la partie solide est un résidu usé de rafles de maïs;
Étape 4: effectuer successivement, sur l'hydrolysat de rafles de maïs, un traitement de neutralisation de la cuve de neutralisation du filtrat, un traitement de décoloration du séparateur à membrane de nanofiltration, un traitement de désalinisation du séparateur d'électrodialyse, un traitement de concentration de la cuve de concentration par évaporation, un traitement de cristallisation de la cuve de cristallisation, et un traitement de séparation solide-liquide du séparateur centrifuge afin d'obtenir respectivement un xylose cristallisé et une liqueur mère de xylose, effectuer un traitement de séchage du sécheur sur le xylose cristallisé afin d'obtenir un produit fini cristallin de xylose premium, transporter la liqueur mère de xylose vers la cuve de mélange de solution sucrée par une conduite; dans lequel une pureté du produit fini cristallin de xylose premium est supérieure à 99%, et une transmittance lumineuse du produit fini cristallin de xylose premium est supérieure à 98%; et
Étape 5: transporter le résidu usé de rafles de maïs successivement vers la cuve de neutralisation des résidus de filtration pour un traitement de neutralisation puis vers le réacteur d'hydrolyse enzymatique pour un traitement d'hydrolyse enzymatique afin d'obtenir une solution enzymatique, transporter la solution enzymatique vers la cuve de mélange de solution sucrée par une conduite pour être mélangée avec la liqueur mère de xylose afin d'obtenir une solution de mélange, puis transporter la solution de mélange à travers un traitement de réaction de brunissement du réacteur de brunissement, un traitement d'évaporation de la cuve flash, un traitement de séparation du séparateur à membrane d'ultrafiltration, et un traitement d'évaporation à film raclé de l'évaporateur à film raclé afin d'obtenir un produit de pigment caramel haut de gamme; dans lequel un indice de couleur du pigment caramel haut de gamme atteint 50000 EBC.

6. Le procédé selon la revendication 5, dans lequel, à l'Étape 1, une concentration en pourcentage massique de sucres secs dans les rafles de maïs est dans une plage de 60-70 wt%, les sucres secs contenant des sucres hexoses de 50-55 wt% et des sucres pentoses de 45-50 wt%; une solution d'acide sulfurique de 0,20-0,30 wt% est utilisée pour le lavage acide, et un rapport liquide/solide pour le lavage acide est de 3:1.

7. Le procédé selon la revendication 5, dans lequel, à l'Étape 2, pendant le traitement d'hydrolyse acide, un rapport de matériau de rafles de maïs à liquide est de 1:5, une teneur ajoutée en acide sulfurique est dans une plage de 1,2-1,5%, une température est dans une plage de 120-125 °C, et une durée est dans une plage de 100-140 min.

8. Le procédé selon la revendication 5, dans lequel, à l'Étape 4, pendant le traitement de décoloration du séparateur à membrane de nanofiltration, une température de fonctionnement du séparateur à membrane de nanofiltration est dans une plage de 40-60 °C, et une pression de fonctionnement est dans une plage de 15-35 bar; et pendant le traitement de désalinisation du séparateur d'électrodialyse, un courant de fonctionnement du séparateur d'électrodialyse est dans une plage de 60-120 A, une tension est dans une plage de 200-250 V, et une pression de fonctionnement est dans une plage de 15-35 bar.

9. Le procédé selon la revendication 5, dans lequel, à l'Étape 5, pendant le traitement d'hydrolyse enzymatique, le pH est ajusté à 5,5-6,5, et une enzyme composite cellulosique commerciale à un pourcentage de 2-4 % est ajoutée en tant que catalyseur, une température de réaction d'hydrolyse enzymatique est contrôlée dans une plage de 40-60 °C, et une durée de réaction d'hydrolyse enzymatique est dans une plage de 72-96 h; pendant le traitement de réaction de brunissement du réacteur de brunissement, un agent auxiliaire de réaction est une combinaison d'eau ammoniacale ou de carbonate d'ammonium, de bicarbonate d'ammonium et d'urée, un rapport de combinaison est dans une plage de 6:2:2-4:4:2,
un procédé de réaction comprend: ajuster d'abord le pH à 7,0-9,0, puis élever la température à 80-90 °C pour réagir pendant 30-50 min, puis élever la température à 160-180 °C pour réagir pendant 3~5 h, puis ajuster le pH à 2-4, puis abaisser la température à 130-150 °C pour réagir pendant 0,5~1 h;
pendant le traitement de séparation du séparateur à membrane d'ultrafiltration, une température de fonctionnement du séparateur à membrane d'ultrafiltration est dans une plage de 40-60 °C, et une pression de fonctionnement est dans une plage de 5-7 bar; et
pendant le traitement d'évaporation à film raclé de l'évaporateur à film raclé, une pression d'évaporation est dans une plage de 4-5 bar, et un degré de vide est dans une plage de 80~ 90 kpa.
